# EUROPEAN PATENT APPLICATION

(11) **EP 4 574 105 A1**
(43) Date of publication of application: **25.06.2025**
(21) Application number: 23218525.6
(22) Date of filing: 20.12.2023
(51) Int. Cl.: A61F 5/50, A61F 5/11

(54) **DEVICE FOR NAIL-BITING HABITS**

(71) Applicant: SRL ONYKO, 1180 Uccle (BE)
(72) Inventor: ESPOSITO, Ethan, 1180 Uccle (BE)
(74) Representative: Torner, Juncosa I Associats, SL

(57) **Abstract**

A device for nail-biting habits is proposed. The device comprises a first portion formed by different mountable pieces, including a ring (10) with biting protheses (11); a central piece (12) with a protrusion to receive the ring (10); a rotatable piece (13) to engage with the protrusion of the central piece (12) to rotate the ring (10) with biting protheses (11); an open box (14) having an opening on a side thereof, the open box (14) being configured to receive the central piece (12) with the ring (10) and the rotatable piece (13) mounted thereon; and a lid (15) with a central hole to close the open box (14). A given biting prothesis of the biting protheses (11) is arranged to exit through the opening of the open box (14) when the rotatable piece (13) is rotated, such that the biting prothesis (11) can substitute a nail-biting habit.

## Description

### Technical Field

The present invention relates to a device for nail-biting habits. Particularly, the device comprises biting prothesis that replicate the appearance of natural nail edges, such as those commonly chewed by individuals afflicted with onychophagia.

### Background of the Invention

Nail-biting is a common habit where individuals chew or gnaw on their fingernails. This behavior is often a response to stress, anxiety, boredom, or nervousness. Nail-biting can lead to a range of issues, including damage to the nails and surrounding skin, the risk of infection, and dental problems if it involves biting the nails with the teeth.

Onychophagia, on the other hand, is a more severe form of nail-biting. It's considered a compulsive behavior and is often categorized as an obsessive-compulsive disorder (OCD). People with onychophagia may find it extremely challenging to stop biting their nails, even if they want to.

Both nail-biting and onychophagia can have negative effects on a person's physical and psychological well-being, and so, individuals who struggle with these habits may seek various methods or tools to help them quit.

One of such nail-biting quitting solutions is proposed in EP2358232-A1, which relates to the use of prepregs for nailtips consisting of glass fiber cloth and preformed for different kinds of nailbeds. The front tips can be easily cut to a desired shape, thus, matching individual shape and taste.

Another solution is known by WO201329137-A1, which provides a device for protecting nails to prevent users who suffer from onychophagy from biting their nails. The device can be produced in different materials, has a circular base, is hollow within and has an anatomical, moldable loop at the end of the finger, which protects the user's nail and cuticle.

Likewise, US20060219251-A1 discloses a system for reducing or stopping the undesirable behavior of nail biting. The system comprises a prosthesis assembly including a prosthesis body maintaining a prompting device electronically connected to a switch; and an attachment device coupled to the prosthesis body and configured for attaching the prosthesis assembly to a user.

Notwithstanding the existing remedies, there is a pressing need for new and alternative solutions to tackle the issue of nail-biting.

### Description of the Invention

To that end, an object of the present invention is to provide a device to enable individuals to substitute their nail-biting habit with a healthier alternative.

This object is fulfilled by a device with the characteristics of claim 1.

The present invention proposes a device for nail-biting habits, comprising a first portion formed by different mountable pieces, including a ring with biting protheses; a central piece with a protrusion to receive the ring; a rotatable piece configured to engage with the protrusion of the central piece to rotate the ring with biting protheses; an open box having an opening on a side thereof, the open box being configured to receive the central piece with the ring and the rotatable piece mounted thereon; and a lid with a central hole to close the open box.

In the proposed device, a given biting prothesis of the biting protheses is arranged to exit through the opening of the open box when the rotatable piece is rotated, such that the biting prothesis can be used as a substitute for a nail-biting habit.

In some embodiments, the device further comprises a second portion to gather the first portion with the different mountable pieces mounted thereon.

In some embodiments, the second portion comprises a C-shaped form.

In some embodiments, the device is made of plastic material.

In some embodiments, the ring comprises between eight and twenty biting protheses.

In some embodiments, at least part of the side surface of the protrusion of the central piece is corrugated.

### Brief Description of the Drawings

The previous and other advantages and features will be more fully understood from the following detailed description of embodiments, with reference to the attached figures, which must be considered in an illustrative and non-limiting manner, in which:
Fig. 1 is an exploded view of the elements that can constitute the proposed device, according to an embodiment of the present invention.
Fig. 2 shows the assembly of the rotatable piece with the central piece and the ring with biting protheses.
Fig. 3 shows the complete assembly of all the pieces illustrated in Fig. 1.

### Detailed Description of the Invention and of Preferred Embodiments

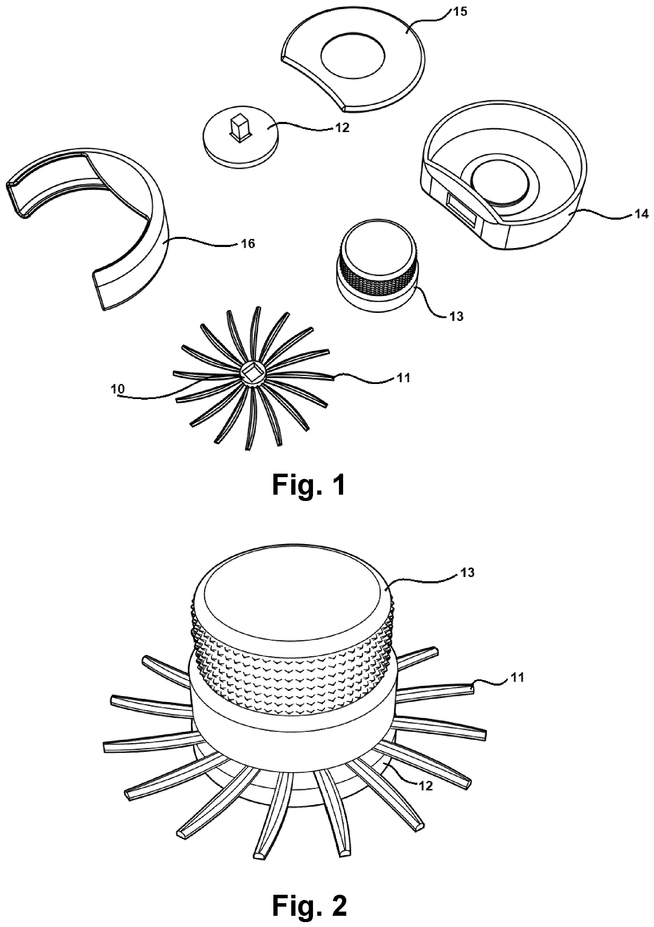

Figs. 1-3 show an embodiment of the proposed device 1 to be used as a substitute for a nail-biting habit.

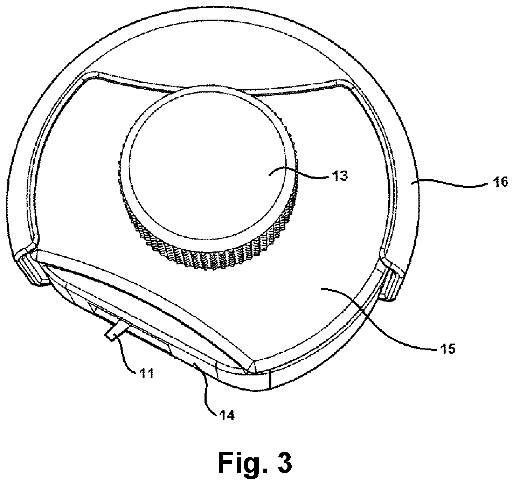

In this particular embodiment, the device 1 consists of two main portions: a first portion, comprising a ring 10 equipped with a variable number of biting prostheses 11 (e.g. eight, ten, fifteen, twenty, etc.), a central piece 12, a rotatable piece 13, an open box 14, and a lid 15 with a central hole, and a C-shaped second portion 16 responsible for securing and enclosing the first section, along with the aforementioned elements, as illustrated in Fig. 3. Particularly, the different elements of the device 1 are made of plastic material or the like.

It's worth noting that in alternative embodiments, which are not illustrated, the device 1 may omit the second section 16. Likewise, the second portion can comprise other shapes.

The central piece 12 features a protrusion, serving the purpose of accommodating both the ring 10 and the rotatable piece 13, as illustrated in Fig. 2. The open box 14 has an opening on one of its sides and is disposed to receive the central piece 12 with the ring 10 and the rotatable piece 13 assembled.

Specifically, the rotatable piece 13 is engineered to engage with the protrusion, allowing for the rotation of the ring 10 along with its associated biting prostheses 11. In other words, with each rotation of the rotatable piece, a specific biting prosthesis 11 can extend through the open box's opening, enabling a user to bite it instead of their own nails.

In the illustrated embodiment, part of the side surface of the rotatable piece 13 is textured for better gripping during rotation.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible.

The scope of the present invention is defined in the following set of claims.

## Claims

1. A device for nail-biting habits, comprising:
a first portion formed by different mountable pieces, including:
a ring (10) with biting protheses (11);
a central piece (12) with a protrusion to receive the ring (10);
a rotatable piece (13) configured to engage with the protrusion of the central piece (12) to rotate the ring (10) with biting protheses (11);
an open box (14) having an opening on a side thereof, the open box (14) being configured to receive the central piece (12) with the ring (10) and the rotatable piece (13) mounted thereon; and
a lid (15) with a central hole configured to close the open box (14),
wherein a given biting prothesis of the biting protheses (11) is arranged to exit through the opening of the open box (14) when the rotatable piece (13) is rotated, such that the biting prothesis (11) can be used as a substitute for a nail-biting habit.

2. The device of claim 1, further comprising a second portion (16) configured to gather the first portion with the different mountable pieces mounted thereon.

3. The device of claim 2, wherein the second portion (16) comprises a C-shaped form.

4. The device of any one of the previous claims, wherein it is made of plastic material.

5. The device of any one of the previous claims, wherein the ring (10) comprises between eight and twenty biting protheses (11).

6. The device of any one of the previous claims, wherein at least part of a side surface of the protrusion of the central piece (12) is corrugated.
